(19) Europäisches Patentamt

European Patent Office

Office européen des brevets

(11) **EP 1 054 006 A1**

(12) **EUROPEAN PATENT APPLICATION**

(43) Date of publication:
22.11.2000 Bulletin 2000/47

(51) Int Cl.$^7$: **C07D 251/60**

(21) Application number: 99201597.4

(22) Date of filing: 20.05.1999

| (84) Designated Contracting States:<br>AT BE CH CY DE DK ES FI FR GB GR IE IT LI LU MC NL PT SE<br>Designated Extension States:<br>AL LT LV MK RO SI | (71) Applicant: DSM N.V.<br>6411 TE Heerlen (NL)<br><br>(72) Inventor: Tjioe, Tjay Tjien<br>6136 BR Sittard (NL) |

(54) **Method for preparing melamine**

(57)  Method for preparing melamine from urea via a high-pressure process in which solid melamine is obtained by transferring the melamine melt coming from the reactor to an expansion vessel where the melamine melt is cooled by incorporated ammonia. The melamine melt coming from the reactor is fed into a gas/liquid separator where additional ammonia is added to remove oxygen-containing compounds. The purified melamine melt is then fed into a vaporizer where the melamine melt is vaporized. The melamine vapour, along with ammonia gas, is then fed into a quench vessel where the melamine vapour is cooled and solidified by mixing with a cooling fluid such as liquid ammonia. The melamine vapour is thereby directly converted into melamine powder after which the melamine powder is cooled further and the ammonia pressure is released.

EP 1 054 006 A1

Printed by Jouve, 75001 PARIS (FR)

## Description

The invention relates to a method for preparing melamine from urea via a high-pressure process in which solid melamine is obtained by transferring a melamine melt to an evaporator vessel from which melamine vapour is extracted and cooled in a quench vessel to produce solid high purity melamine.

Various methods for the production of melamine have been described in previous publications including, inter alia, WO 95/01345 which describes a high-pressure process for preparing melamine from urea. In particular, WO 95/01345 describes how urea is pyrolyzed in a reactor having an integral gas/liquid separator, the resulting melamine melt is then fed into a vaporizer to generate melamine vapour, and the melamine vapour is fed into a quench vessel and cooled to produce melamine.

According to WO 95/01345 the reactor operates at a pressure of from 5 to 15 MPa and a temperature of from 360 to 430°C, to produce a reactor product comprising primarily molten melamine and byproduct gases $CO_2$ and $NH_3$. The byproduct gases generated in the reactor including $CO_2$, $NH_3$ and melamine vapour are separated from the melamine melt and removed from the reactor. The reactor offgas reportedly comprises 0.9% melamine, 14.9% $CO_2$, with the balance being $NH_3$.

In the vaporizer, the melamine melt is vaporized by reducing the pressure, increasing the temperature, feeding ammonia gas into the vaporizer, or by a combination of these methods. As described in WO 95/01345, the vaporizer operates at a temperature between 420 and 470°C at a pressure of 2 to 9 MPa. The melamine vapour produced is then transferred to a quench vessel. According to WO 95/01345, when the vaporizer is operated at 450°C and 5 MPa, for each 1 kg of melamine a minimum of 2.4 kg of ammonia must be added to the vaporizer. Operated in this manner, the residence time in the vaporizer reportedly has no significant effect and the vaporizer out gas comprises 3.1 mole% melamine.

In the quench vessel the melamine vapour is directly contacted with a cooling liquid, the temperature of the cooling liquid being less than 130°C, under a pressure of less than 4 MPa to solidify the melamine vapour and thereby recover melamine having 99.9% purity. In the example provided for instance, the quench vessel was operated at 120°C and 0.2 MPa (absolute) and the melamine and ammonia gas was quenched with water. To the extent that the water is not completely evaporated, the remainder will be discharged with and later removed from the solidified melamine. The ammonia vapour from the quench vessel will be condensed and recycled.

The importance of removing the $CO_2$ produced in the reactor from the melamine melt prior to vaporization is recognized in WO 95/01345. According to WO 95/01345, the gas/liquid separator function of the reactor itself will remove sufficient $CO_2$ in the offgases to avoid introducing $CO_2$ into the vaporizer. WO 95/01345 acknowledges that some $CO_2$ will pass into the vaporizer but contends that the amount is insignificant.

Relying solely on the gas/liquid separator function of the reactor to remove $CO_2$, however, does not address the presence of additional oxygen-containing compounds in the melamine melt. The failure to provide a means for the removal of other oxygen-containing compounds from the melamine melt is, therefore, a disadvantage of the WO 95/01345 process.

Applicant has found that if such oxygen-containing impurities are not removed, in particular ammelide, ammeline, and ammonium (iso)cyanate, they will produce $CO_2$ according to the following reactions:

$$ammeline + NH_3 \rightarrow melamine + H_2O$$

$$ammelide + 2NH_3 \rightarrow melamine + 2H_2O$$

$$H_2O + NH_4\text{-}OCN \rightarrow 2NH_3 + CO_2$$

The $CO_2$ formed will be transported with the melamine and ammonia to the quench section. In the quench section the $CO_2$ produced by these reactions, in addition to any $CO_2$ already present in the melamine melt, may, in turn, react with the excess ammonia to form, under the circumstances present in the quench section, ammonium carbamate according to the reaction:

$$CO_2 + 2NH_3 \rightarrow ammonium\ carbamate$$

Economically, recycling all or most of the gas stream coming from the quench vessel is desirable, but such recycling tends to accumulate $CO_2$ resulting in fouling in the filters and the ammonia condensors caused by ammonium carbamate formation.

Research by applicant has also found that it is generally desirable to operate the vaporizer at very high temperatures, preferably above 420 °C, however maintaining temperatures this high creates practical problems in the vaporizer. In order to achieve higher temperatures in the melamine melt and more rapid vaporization in this manner, the temperature of the heating fluid must be maintained at an even higher temperature, typically above 455°C. Operation at these elevated temperatures in combination with relatively low operating pressures (typically below 9 MPa as mentioned in WO 95/01345), however, will result in fouling of the heating surface on the process side resulting in a reduced production capacity of the vaporizer. The fouling is caused by decomposition of melamine at high temperatures on the surface of the heating elements of the vaporizer as has been noted in US 3,484,440. In addition the operational life of the heat transfer elements will decrease as has been noted in WO 95/01345.

The object of the present invention is to provide an improved method for preparing melamine from urea, in which melamine is obtained directly from melamine vapour as a dry powder having a high degree of purity. More particularly, the object of the present invention is to obtain an improved high-pressure process for preparing melamine from urea, in which oxygen-containing by-products including $CO_2$, $H_2O$, ammelide, ammeline, and ammonium isocyanate, are removed from the melamine melt before it enters the vaporizer. The improved purity of the melamine melt fed into the vaporizer reduces the production of $CO_2$ and, consequently, reduces the formation of ammonium carbamate in the quench vessel, thereby providing for increased gas recycling and improved performance of $NH_3$ condensor and filters and melamine yields.

Another object of the present invention is that higher temperature vaporizer operation can be maintained more consistently by modifying the vaporizer construction and operation in such a manner that the temperature of the heating surface which is in contact with the melamine melt is reduced without reducing the capacity of the vaporizer. By reducing the surface temperature decomposition of melamine is prevented and no fouling occurs.

The applicant has found that high purity melamine can be produced continuously and consistently from urea via a high-pressure process, characterized in that a melamine melt that includes oxygen-containing compounds is transferred into a stripper, where, under the addition of 0.01-10 moles of ammonia per mole of melamine, preferably 0.02-5 moles of ammonia per mole of melamine, the oxygen-containing compounds are removed to a level below 0.2 wt%, preferably below 0.05 wt%, to produce a purified melamine melt, the purified melamine melt being fed into a vaporizer to produce melamine vapour, the melamine vapour being fed into a quench vessel and there mixed with a cooling fluid to produce melamine having purity of better than 99%. The stripper is operated at a pressure between 5 and 25 MPa, preferably 5 and 15 MPa, and a temperature between 335 and 450°C, preferably between 350 and 425°C.

In a preferred embodiment the invention also relate to a process for the production of melamine wherein the melamine melt is evaporated in an evaporator which contains heating elements which are in contact with the melamine melt with a temperature within the range 410 - 480 °C, whereby along these heating elements excess ammonia gas is purged resulting in a gas-liquid mixture flowing along the heating elements, wherein the superficial gas velocity passing the heating elements is larger than 0.02 m/s, preferably larger than 0.05 m/s and the quantity of excess gaseous ammonia added to the vaporizer is larger than 0.2 kg per kg of melamine, preferably between 0.5 - 5 kg per kg of melamine. The ammonia gas can also be fed into the evaporator in combination with the melamine melt. The superficial velocity is the volumetric gas flow (in $m^3$/s) divided by the free cross sectional area of the gas-liquid mixture (in $m^2$) between the heating elements.

In a further embodiment the ammonia gas enters the vaporizer below the heating elements and the gas passes along vertically placed heating surface (e. g. tubes). The ammonia flows vigorously through the melamine melt along the surface of the tubes as bubbles. These bubbles significantly increase the heat tranfer from the tubes to the melamine melt through forced convection. This improved heat transfer permits the melamine melt to be vaporized at higher temperatures than permitted by the prior art without unacceptable increases in the tube surface temperatures. By the turbulence caused by the two-phase flow the surface of the heating elements is cooled strongly compared with the situation without the ammonia gas purge. So the surface temperature of the heating elements will be lowered and no decomposition of the melamine will occur.

The advantage of the method according to the present invention is the continuous production, on a commercial scale, of dry melamine powder having a purity above 98.5 wt%, and generally above 99 wt%, that has very good color and surface area characteristics. The higher vaporizer temperatures permitted by the present method provide increased capacity over prior art vaporizers while maintaining acceptable lifetimes for the vaporizer tubes. The high purity melamine produced according to the present invention is suitable for virtually any melamine application, including melamine-formaldehyde resins used in laminates and/or coatings.

The preparation of melamine preferably uses urea as the raw material, the urea being fed into the reactor as a melt and reacted at elevated temperature and pressure. Urea reacts to form melamine, and the by-products $NH_3$ and $CO_2$, according to the following reaction equation:

$$6 \, CO(NH_2)_2 \rightarrow C_3N_6H_6 + 6 \, NH_3 + 3 \, CO_2$$

The production of melamine from urea can be carried out at high pressure, preferably between 5 and 25 MPa, without the presence of a catalyst, at reaction temperatures between 335 and 450°C, and preferably between 350 and 425°C. The by-products $NH_3$ and $CO_2$ are usually recycled to an adjoining urea factory.

The above-mentioned objective of the invention is achieved by employing an apparatus suitable for the preparation of melamine from urea. An apparatus suitable for the present invention may comprise a scrubber unit, a reactor with or without an integrated gas/liquid separator or a separate gas/liquid separator, a stripper, a vaporizer, a quench vessel, and possibly additional cooling vessels.

In one embodiment of the invention, melamine is prepared from urea in an apparatus comprising a scrubber, a melamine reactor with an integrated gas/liquid separator, a separate gas/liquid separator with an integrated stripper, a vaporizer, and a quench vessel. In this embodiment, the urea melt is fed into a scrubber operating at a pressure of from 5 to 25 MPa, preferably from 8 to 20 MPa, and at a temperature above the melting point of urea. This scrubber may be provided with a cooling jacket or internal cooling bodies to provide additional temperature control.

As it passes through the scrubber, the urea melt contacts the reaction waste gases coming from the reactor and/or the gases coming from the separate gas/liquid separator. The reaction waste gases from both the reactor and the separate gas/liquid separator mainly consist of $CO_2$ and $NH_3$ and may include melamine vapour. The urea melt scrubs the melamine vapour from the $CO_2$ and $NH_3$ waste gases and carries this melamine along back to the reactor. In the scrubbing process, the waste gases are cooled from the temperature of the reactor, *i.e.*, from 350 to 425°C, to from 170 to 240°C, the urea being heated to from 170 to 240°C. The $CO_2$ and $NH_3$ waste gases are removed from the top of the scrubber and may, for example, be recycled to an adjoining urea factory, where they can be used as raw materials for the urea production.

The preheated urea melt is drawn off from the scrubber, together with the melamine scrubbed from the waste gases, and transferred to the high pressure reactor operating at pressures between 5 and 25 MPa, and preferably between 8 and 20 MPa. This transfer may be achieved using a high-pressure pump or, where the scrubber is positioned above the reactor, gravity, or a combination of gravity and pumps.

In the reactor, the urea melt is heated to a temperature between 335 and 450°C, preferably between about 350 and 425°C, under a pressure between 5 and 25 MPa, preferably between 8 and 20 MPa, to convert the urea into melamine, $CO_2$, and $NH_3$. In addition to the urea melt, a certain amount of ammonia can be metered into the reactor as, for example, a liquid or hot vapour. The additional ammonia, although optional, may serve, for example, to prevent the formation of byproducts of melamine such as melam, melem, and melon, or to promote mixing in the reactor. The amount of additional ammonia supplied to the reactor may be up to 10 moles ammonia per mole of urea, preferably up to 5 moles ammonia per mole of urea, and, most preferably, up to 2 moles of ammonia per mole of urea.

The bulk of the $CO_2$ and $NH_3$ produced during the melamine formation reaction, as well as any additional ammonia supplied to the reactor, collect in the reactor's integrated separation section, for example in the top of the reactor, and be removed from the melamine melt or can be separated in a separate gas/liquid separator. In an embodiment the crude melamine melt is then removed from the reactor or gas/liquid separator and fed into the downstream stripper where additional ammonia will be added. In an embodiment the stripper can be combined with the gas/liquid separator in one vessel. The pressure and temperature in the stripper can differ from the temperature and/or the pressure of the reactor. The pressure in the stripper is in general between 5 and 25 MPa, preferably between 5 and 15 MPa. The stripper can be equipped with heating elements to increase the temperature of the melamine melt to improve the stripping proces. The temperature in the stripper is in general between 335 and 450°C, preferably between 350 and 425°C. The amount of ammonia added to the stripper in this case should be 0.01-10 moles of ammonia per mole of melamine, and preferably 0.02-5 moles of ammonia per mole of melamine. The ammonia added to the stripper both promotes the rapid separation of carbon dioxide from the reactor product, thus preventing the formation of additional oxygen-containing byproducts, and promotes the decomposition of any oxygen-containing byproducts that formed in the reactor. As described above, the gas mixture removed from the stripper may then be passed to the scrubber in order to remove melamine vapour and preheat the urea melt.

The purified melamine melt, typically having a temperature between the melting point of melamine and 450°C, is then drawn off from the stripper and fed into the vaporizer. In the vaporizer vessel, the purified melamine melt is heated to a temperature generally in between 410 and 480°C, preferably between 425 and 470°C, under an ammonia pressure of between 2 and 15 MPa, preferably between 3 and 10 MPa, to vaporize all of the melamine melt.

In a configuration, a vaporizer according to the present invention may contain the melamine melt passing through the vaporizer in a series of smaller tubes, the tubes being partly or totally submerged in the melamine melt. The tubes, and their associated ammonia injection device, are configured so that ammonia bubbles flow vigorously and co-currently through the melamine melt along the inner surface of the tubes (pool evaporation). A central large draught tube (without ammonia gas injection) is placed between the smaller tubes and the melamine melt recirculates through this central draught tube.

In either instance, the vaporization vessel includes sufficient thermal heat transfer capacity to continuously vaporize all of the melamine melt feed at the desired temperatures. The total amount of excess gaseous ammonia added to the vaporizer should generally be proportional to the melamine melt feed in the range of 0.2 - 20 kg/kg melamine,preferably between 0.5 - 5 kg ammonia is used per kg of melamine melt feed. The superficial velocity of the gaseous ammonia, passing the heating elements of the vaporizer, is for example between 0.02 - 5 m/s, preferably between 0.05 - 1.5 m/s.

The ammonia gas can also be fed into the evaporator in combination with the melamine melt. The superficial velocity is the volumetric gas flow (in $m^3/s$) divided by the free cross sectional area of the gas-liquid mixture (in $m^2$) between the heating elements. Operating the vaporizer according to the present invention the gas removed from the vaporizer will contain 5-50 w% of melamine.

The vaporizer may include a plurality of heating elements through which a heating fluid, typically a molten salt, is passed to heat the melamine. Alternatively, the melamine melt itself may be passed through the vaporizer through a series of heating elements that are surrounded by the heating fluid. The vaporizer may include a plurality of tubes through which the melamine melt and the ammonia gas are passed and the tubes are heated from the outside of the tubes. Alternatively the melamine melt and the ammonia gas may surround a series of tubes which are heated from the inside of the tubes. Heating can be performed with a fired furnace, electrically, or with a liquid heating medium e.g. molten salt. In either instance, the vaporizer includes sufficient thermal transfer capacity to vaporize all of the melamine melt feed.

The melamine and ammonia vapours from the improved vaporizer are then fed into the quench vessel and cooled to produce melamine. The melamine vapour is cooled with a fluid cooling agent such as water, an aqueous ammonia solution, liquid ammonia, or cool ammonia gas, preferably liquid ammonia. The ammonia gas removed from the quench chamber may then be recycled.

Additional cooling of the solidified melamine may be effected through the addition of cool ammonia gas or liquid ammonia, separately or in combination with mechanical agitation and indirect cooling through contact with cooled surfaces. Examples of means for mechanically agitating the melamine powder include a screw and rotating drum, a rotating bowl, rotating discs, rotating segmented discs, rotating pipes and the like.

The invention will be explained in more detail with reference to the following example and comparative example.

Example I:

0.4 kg of melamine melt containing 1.8 wt% oxygen containing compounds ($CO_2$, urea, ammonium isocyanate, ammeline and ammelide) was transferred to a stripper/evaporator. The melamine melt was stripped with 0.12 kg of ammonia at a temperature of 408 °C and a pressure of 8.7 MPa.

Then the temperature was raised to 450 °C and the melamine melt was evaporated with the addition of 1.1 kg ammonia at 5.2 MPa.

The superficial gas velocity of the gaseous ammonia in the vaporizer is 0.1 m/s.

The vaporizer has 12 vertically placed tubes with a length of 1 m each and an inner diameter of 18 mm. The melamine liquid level is kept above the tubes (pool evaporator) and the tubes are heated from the outside with molten salt with a temperature of 475 °C resulting in an melamine melt temperature of 450 °C in the evaporator.

Hot ammonia gas of 438 °C is injected in the melamine melt at a position below the tubes and a gas-liquid mixture flows upwards through the tubes and the liquid recirculates through a central draught tube. The evaporator has a central draught tube with an inner diameter of 55 mm and ammonia gas is prevented to enter the draught tube from below. The production rate of the vaporizer is 0.10 kg/min of melamine.

The melamine vapour was quenched with liquid ammonia in a quench vessel at 3.2 MPa and 68 °C. The quench vessel was depressurized and the total content (gas + solid) was analyzed for oxygen containing compound. No $CO_2$ and ammonium carbamate could be detected (less than 0.03 g).

The purity of the melamine was 99.2%.

Comparative example A:

The same experiment as described in example I was performed except that the stripping procedure with ammonia was skipped. Then the hot ammonia gas is bubbled through the melamine melt above the tubes to transport the evaporated melamine to the quench vessel. The production rate of the vaporizer is 0.07 kg/min of melamine. The quench vessel was depressurized and analyzed. Oxygen containing compounds among others 2 g of $CO_2$ and ammonium carbamate were found in the vessel.

These compounds would cause scaling of the condensers in a continuous process operation with recirculation of the ammonia. The purity of the melamine was 98.7%.

**Claims**

1. A method for preparing melamine from urea via a high-pressure process, characterized in that a melamine melt that includes oxygen-containing compounds is transferred into a stripper, where, under the addition of 0.01-10 moles of ammonia per mole of melamine, the oxygen-containing compounds are removed to a level below 0.2 wt%, to produce a purified melamine melt, the purified melamine melt being fed into a vaporizer to produce melamine vapour, the melamine vapour being fed into a quench vessel and there mixed with a cooling fluid to produce melamine having purity of better than 99%.

2. A method according to claim 1, characterized in that the oxygen-containing compounds are removed to a level below 0.05 wt%.

**3.** A method according to claim 1, characterized in that the the addition of ammonia in the stripper is 0.02-5 moles per mole of melamine.

**4.** A method according to claim 1, characterized in that the stripper is operated at a pressure between 5 and 15 MPa and a temperature between 350 and 425 °C.

**5.** Method according to claims 1 - 4, characterized in that the evaporator contains heating elements which are in contact with the melamine melt with a temperature within the range 410 - 480 °C, whereby along these heating elements excess ammonia gas is purged resulting in a gas-liquid mixture flowing along the heating elements, wherein the superficial gas velocity passing the heating elements is larger than 0.02 m/s and the quantity of excess gaseous ammonia added to the vaporizer is larger than 0.2 kg per kg of melamine.

**6.** Method according to claim 5, characterized in that the vaporizer is operated at a pressure between 3 and 10 MPa and a temperature between 425 and 470°C.

**7.** Method according to claim 5, characterized in that the quantity of the excess gaseous ammonia is between 0.5 - 5 kg per kg melamine melt feed.

**8.** Method according to claim 5, characterized in that the superficial velocity of the gaseous ammonia, passing the heating elements of the vaporizer is between 0.05 m/s - 1.5 m/s.

**9.** Method according to claim 5, characterized in that the excess gaseous ammonia enters the vaporizer below the heating elements and the gas passes along the vertically placed heating surface.

**European Patent Office**

## EUROPEAN SEARCH REPORT

Application Number

EP 99 20 1597

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | EP 0 808 836 A (DSM NV) 26 November 1997 (1997-11-26) * column 6, line 5 - line 29 * | 1 | C07D251/60 |
| Y,D | WO 95 01345 A (KEMIRA OY ;TURUNEN ILKKA (FI); OINAS PEKKA (FI)) 12 January 1995 (1995-01-12) * claim 1 * | 1 | |
| A | WO 96 20182 A (AGROLINZ MELAMIN GMBH) 4 July 1996 (1996-07-04) * claim 1 * | 1 | |
| A | US 3 116 294 A (G. MARULLO ET AL) 31 December 1963 (1963-12-31) * claim 1 * | 1 | |

|  |  |
|---|---|
|  | TECHNICAL FIELDS SEARCHED (Int.Cl.7) |
|  | C07D |

The present search report has been drawn up for all claims

| Place of search | Date of completion of the search | Examiner |
|---|---|---|
| THE HAGUE | 11 October 1999 | De Jong, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                     

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 99 20 1597

This annex lists the patent family members relating to the patent documents cited in the above-mentioned European search report.
The members are as contained in the European Patent Office EDP file on
The European Patent Office is in no way liable for these particulars which are merely given for the purpose of information.

11-10-1999

| Patent document cited in search report | | Publication date | Patent family member(s) | | Publication date |
|---|---|---|---|---|---|
| EP 0808836 | A | 26-11-1997 | NL 1003105 | C | 18-11-1997 |
| | | | CN 1170720 | A | 21-01-1998 |
| | | | JP 10045730 | A | 17-02-1998 |
| WO 9501345 | A | 12-01-1995 | FI 933033 | A | 02-01-1995 |
| | | | EP 0706519 | A | 17-04-1996 |
| | | | JP 8512042 | T | 17-12-1996 |
| | | | US 5731437 | A | 24-03-1998 |
| WO 9620182 | A | 04-07-1996 | AT 402294 | B | 25-03-1997 |
| | | | AT 239294 | A | 15-08-1996 |
| | | | AT 176228 | T | 15-02-1999 |
| | | | AU 692034 | B | 28-05-1998 |
| | | | AU 4386196 | A | 19-07-1996 |
| | | | BG 101594 | A | 27-02-1998 |
| | | | BR 9510363 | A | 23-12-1997 |
| | | | CA 2207059 | A | 04-07-1996 |
| | | | CN 1171102 | A | 21-01-1998 |
| | | | DE 59505010 | D | 11-03-1999 |
| | | | EP 0799212 | A | 08-10-1997 |
| | | | ES 2128798 | T | 16-05-1999 |
| | | | HR 950607 | A | 31-10-1997 |
| | | | HU 77060 | A | 02-03-1998 |
| | | | JP 10511368 | T | 04-11-1998 |
| | | | NO 972869 | A | 14-08-1997 |
| | | | NZ 298387 | A | 29-04-1999 |
| | | | PL 320789 | A | 27-10-1997 |
| | | | SK 79297 | A | 08-10-1997 |
| | | | TR 960596 | A | 21-07-1996 |
| | | | US 5721363 | A | 24-02-1998 |
| | | | ZA 9510900 | A | 24-06-1996 |
| US 3116294 | A | | NONE | | |

EPO FORM P0459

For more details about this annex : see Official Journal of the European Patent Office, No. 12/82